# EUROPEAN PATENT SPECIFICATION

(11) **EP 2 917 751 B1**
(45) Date of publication and mention of the grant of the patent: **21.04.2021**
(21) Application number: 13798395.3
(22) Date of filing: 04.11.2013
(51) Int. Cl.: G01R 33/563, G01R 33/34, G01R 33/3415, G01R 33/36

(54) **RHEOLOGY UNIT FOR MRI WITH INTEGRATED OSCILLATOR AND RF ANTENNAS**
RHEOLOGIEEINHEIT FÜR MRT MIT INTEGRIERTEM OSZILLATOR UND HF-ANTENNEN
UNITÉ DE RHÉOLOGIE POUR IRM COMPRENANT UN OSCILLATEUR ET DES ANTENNES RF INTÉGRÉS

(30) Priority: 12.11.2012 US 201261725065 P
(43) Date of publication of application: 16.09.2015
(73) Proprietor: Koninklijke Philips N.V., 5656 AG Eindhoven (NL); Philips GmbH, 20099 Hamburg (DE)
(72) Inventor: WIRTZ, Daniel, NL-5656 AE Eindhoven (NL); VERNICKEL, Peter, NL-5656 AE Eindhoven (NL); MAZURKEWITZ, Peter, 5656 AE Eindhoven (DE); LEUSSLER, Christoph, NL-5656 AE Eindhoven (NL)
(74) Representative: Cohen, Julius Simon
(86) International application number: PCT/IB2013/059876
(87) International publication number: WO 2014/072895

(56) References cited:
- US-A1- 2003 065 267
- US-A1- 2010 045 289
- US-A1- 2010 049 029
- US-A1- 2012 010 497
- US-B2- 7 307 423
- LATTA P ET AL: "Convertible pneumatic actuator for magnetic resonance elastography of the brain", MAGNETIC RESONANCE IMAGING, ELSEVIER SCIENCE, TARRYTOWN, NY, US, vol. 29, no. 1, 1 January 2011 (2011-01-01), pages 147-152, XP027554282, ISSN: 0730-725X [retrieved on 2010-12-09]
- UTAROH MOTOSUGI ET AL: "Effects of gadoxetic acid on liver elasticity measurement by using magnetic resonance elastography", MAGNETIC RESONANCE IMAGING, ELSEVIER SCIENCE, TARRYTOWN, NY, US, vol. 30, no. 1, 4 August 2011 (2011-08-04) , pages 128-132, XP028336239, ISSN: 0730-725X, DOI: 10.1016/J.MRI.2011.08.005 [retrieved on 2011-08-17]

## Description

### FIELD OF THE INVENTION

The invention relates to the field of magnetic resonance (MR) rheology imaging. In particular, the invention relates to the field of rheology modules for use in a MR rheology imaging system, whereby the rheology module is adapted to introduce mechanical oscillations into a subject of interest. Furthermore, the invention relates to the field of rheology arrangements for use in a MR rheology imaging system, comprising at least one RF antenna module comprising at least one RF antenna unit, and at least one rheology module as specified above. Still further, the invention relates to the field of MR rheology imaging systems comprising a rheology module as specified above.

### BACKGROUND OF THE INVENTION

In the area of magnetic resonance (MR) imaging, MR rheology is a technique for gathering additional information on tissue properties, which is not accessible with MR imaging alone. Parameters like tissue viscosity or elasticity can otherwise only be determined using biopsy and/or histology. On the other hand it has been demonstrated, that these tissue properties can help in the detection of cirrhotic or cancerous changes, e.g. in liver, breast or brain tissue. In particular, MR rheology has been proven to be especially useful for determining and staging liver cirrhosis as well as breast cancer. Initial applications to degenerative brain diseases have also been proposed.

Typically a MR rheology setup contains a rheology module for introducing mechanical oscillations into a subject of interest and an MR imaging system. In order to determine tissue properties of a particular region of interest (ROI) of the subject of interest, the rheology module is placed close to this area to introduce the oscillations. Appropriate radio frequency (RF) antenna units comprising one or more RF coils are provided for generating the anatomical 'background' images. These RF antenna units can be part of the MR imaging system, or additional RF antenna units can be provided in RF antenna modules to improve the imaging in respect to the ROI of the subject of interest. Particularly, the RF antenna modules are provided close to the ROI.

For some MR measurements, a rheology arrangement is used. The arrangement comprises a rheology module and typically at least one RF antenna module comprising at least one RF antenna unit. These modules are combined and interconnected, so that the rheology arrangement can be placed at the subject of interest or close to the subject of interest. In general, the closer a RF antenna unit is located to the ROI, the better are the results of the MR rheology imaging process. Due to the size of the modules, placement of the RF antenna units is usually not as close to the ROI as desired. The optimum size of the RF-coil to the ROI is determined by this distance. Thus, large coils can image with good results even from a larger distance. A field-of-view (FOV) of such coils on the other hand is relatively large and may not be appropriate for the MR rheology imaging process. If the ROI is rather small and/or close to the surface of the subject of interest, small receive coils perform better due to reduced noise pickup from the surroundings outside the ROI. Moreover a larger number of coil elements allows for speeding up the imaging process using algorithms like SENSE. With few coil elements such an acceleration in imaging is not possible.

Furthermore, cabling of the rheology module and the RF antenna modules is rather complicated. Apart from signaling lines, also a supply line is a required for providing power to the rheology module as well as the RF antenna module. Cabling needs increase the setup time for performing MR rheology measurements, and can even reduce the imaging performance of the MR rheology system. Accordingly, improvements are desired.

A magnetic resonance elastography (MRE) scan is known from US 7 307 423 B2. The scan is performed using an array of transducers for applying a strain wave to tissues in a region of interest. A calibration process is performed prior to the scan in which the strain wave produced by each transducer in the array is imaged using an MRE pulse sequence so that information may be acquired that enables each transducer to be properly driven during a subsequent MRE scan.

In US application 2003/0065267 A1, a system for generating magnetic resonance imaging (MRI) elastographs of a region in a body is described. The system includes a catheter comprising an elongated member, which is adapted to be inserted into a blood vessel or for insertion into openings in the body, an acoustic transducer positioned near a distal end of the elongated member, and a radio frequency (RF) coil adjacent to the acoustic transducer. The transducer is located near the distal end of the elongated member for transmitting acoustic waves and comprises an inner cylindrical conductive layer surrounding the elongated member, a piezoelectric crystal layer surrounding the inner conductive layer, and an outer cylindrical conductive layer surrounding the piezoelectric crystal layer. To generate an MRI elastograph of a region in a body, the acoustic transducer and the RF coil of the catheter are positioned at the desired region in the body. The acoustic transducer emits acoustic waves in the region of the body, and the RF coil detects RF signals emitted within the region of the body. The detected RF signals are outputted to an MRI receiver to generate the MRI elastograph of the region in the body.

US application 2010/0045289 A1 describes a system for intracorporeal elastography, including an intra-luminal vibratory member configured to be positioned within a lumen of an imaging subject and configured to impart mechanical energy into tissue of the lumen. An acoustic energy source external to the imaging subject is acoustically coupled to the proximal end of a member, and the distal end of the member is adapted to be inserted into a lumen of the subject. The acoustic energy coupled to the member causes at least the distal end of the member to mechanically vibrate and generate shear waves within the subject for use with magnetic resonance elastography. Various acoustic energy sources are described, such as a piezoelectric actuator attached to a proximal end of the vibratory member. In another embodiment, the vibratory member is adapted to include a flexible membrane at its distal end to impart waves into the tissue surrounding the lumen, and comprises a flexible hose. The hose is hermetically coupled to an audio output device at a first end of the hose and at a second end to the distal end of the vibratory member. A pneumatic fluid such as air or other gaseous fluid or a hydraulic fluid is hermetically sealed in the hose. Acoustic energy from the audio output device is transferred to the distal end of the vibratory member through the hose, and application of the time-varying pressure within the distal end of the vibratory member causes the membrane and distal end of the vibratory member to vibrate.

In the article by Peter Latta et al., "Convertible pneumatic actuator for magnetic resonance elastography of the brain", Magnetic Resonance Imaging 29 (2011), p. 147-152, an actuator for brain elastography is described that claims for simplicity. The actuator includes a pair of standard active subwoofers that have been modified with airtight acrylic lids mounted over the speaker membrane and used as a source of air pressure. At the center of the lids, a hose connector has been mounted to attach flexible tubing for the delivery of acoustic waves into passive drivers of a driver unit that consists of standard plastic bottles commonly used as containers for various food and cosmetics products. A homemade fitting is used to connect the containers to the hard-walled flexible tubes.

US application 2012/0010497 A1 describes a unilateral magnetic resonance imaging (MRI) device that is capable of performing magnetic resonance elastography (MRE). The unilateral MRI device includes a magnet assembly that produces a static, polarizing magnetic field extending longitudinally outward from a pole face of the magnet, substantially homogeneous in a transverse plane in the near-field, and varying quasi-linearly along the longitudinal direction away from the pole face. An imaging assembly is fastened over the pole face of the magnet assembly and includes a radio frequency (RF) coil and a magnetic field gradient coil that produces a magnetic field gradient in the near-field along a gradient axis. The imaging assembly includes an annular shaped spacer ring, and a disc-shaped support element extends over the forward pole face of the magnet assembly. The support element has a central opening that houses a disc-shaped, ferromagnetic field shaping element. An annular-shaped magnetic field shaping element is retained against the forward surface of the support element and extends radially inward from the spacer ring to form a circular central bore forward of the ferromagnetic field shaping element.

A set of imaging coils including RF coils and magnetic field gradient coils is mounted within the central bore, forward of the ferromagnetic field shaping element. An MRE transducer element includes an external bending element, a piezoelectric disc disposed within the bore of the annular field shaping element and flush with the forward surface of the annular field shaping element, and a flat piezoelectric extension motor. The piezoelectric disc is positioned beneath the set of imaging coils and beneath an RF shield. The MRE transducer element produces a vibratory motion, or oscillatory stress, in the subject, that provides a phase contrast mechanism by which MRE is performed.

### SUMMARY OF THE INVENTION

It is an object of the invention to provide a rheology module, a rheology arrangement comprising such a module, and a MR rheology imaging system, which enable improved MR rheology imaging, and which facilitate and improve a workflow for performing MR rheology imaging.

In one aspect of the present invention, the object is achieved by a rheology module for use in a magnetic resonance (MR) rheology imaging system as defined in independent claim 1, whereby the rheology module is adapted to introduce mechanical oscillations into a subject of interest, comprising a housing, a mechanical oscillator unit, which is arranged at least partially inside the housing, extends at least partially outside the housing and is movable as a whole relative to the housing, and a transducer for moving the oscillator unit.

With the RF antenna device integrated into the rheology module, an antenna placement close to a region of interest (ROI) can be achieved to improve the MR imaging capabilities of a MR rheology imaging system. Thus, imaging of the ROI can be performed more efficiently. Furthermore, connection and cabling can be facilitated, since only one module has to be connected to generate the oscillation and to operate the RF antenna device. The RF antenna device can be used as transmit/receive or receive only device, where each coil can be a receive coil only or a local transmit/receive coil. The MR rheology module overcomes disadvantages of the separate placement of the mechanical oscillator unit and the RF antenna device. Accordingly, also cable routing can be improved and interaction between mechanical oscillator unit and MR imaging equipment can be reduced. The geometry of the module can be freely chosen. Also the geometry of the RF antenna units and RF coils can be chosen as required. Typically, the RF coils have an essentially circular or rectangular shape. Further preferred, the essentially circular shape can be formed by six to eight linear segments, e.g. in the form of a stop sign or octagon. Preferably, the RF antenna unit is provided at the side of the rheology module, where the mechanical oscillator comes into contact with the subject of interest. Any suitable kind of transducer can be used to generate the oscillation of the mechanical oscillator unit, including an electrical, a pneumatic, or a hydraulic transducer. The electrical transducer is preferably a piezoelectric transducer, which converts electric energy into mechanical energy or an electromechanical transducer making use of the movement of a current driven coil within the static magnetic field of the MR imaging device. Piezoelectric transducers can respond very rapidly to drive voltage changes. In a preferred embodiment, the rheology module is provided with a thin housing, which facilitates its placement. The transducer can be provided as a backpack with a local, heavy mass to provide sufficient mechanical inertia to keep amplitude of the oscillations high. The local mass can be exchanged with respect to a desired application, e.g. for different organs or depending on the thickness of fatty tissue layer. The local mass is chosen to be MR compatible.

According to the invention, at least one RF antenna unit is located on or in the housing. Different placements of the RF antenna unit at the housing are possible. The RF antenna unit is preferably arranged to surround the oscillator unit. The RF antenna unit can be provided at an upper surface of the housing, integrated into the housing, or at an inner surface thereof. Preferably, the RF antenna unit is provided over an entire face of the housing, e.g. the face facing the subject of interest in operation.

According to the invention, at least one RF antenna unit is located on or in the oscillator unit. The RF antenna unit can be provided at an upper surface of the oscillator unit, so that the RF antenna unit is in direct contact with the subject of interest. Alternatively, the RF antenna unit can be integrated into the oscillator unit, or the RF antenna unit can be located at a lower surface thereof facing the housing.

According to a preferred embodiment at least one RF antenna unit comprises a set of multiple RF coils. Multiple RF coils can be used for image generation with increased speed for dynamic processes. The RF coils of the RF antenna unit can be arranged in different ways, e.g. in an array. The RF coils can also be arranged to overlap with adjacent coils and hence being geometrically decoupled from each other. The RF antenna unit can also comprise at least one local, separate transmit coil and a separate receive coil array. For more than one transmit channel, the integrated transmit coil has a nearby RF power combiner to distribute RF power to different transmit coil elements. Accordingly, the MR rheology unit can be used independent from the presence of a large integrated body transmit coil. The receive coil array is detuned during the transmit pulse and the transmit coil is detuned during reception of MRI signals.

According to a preferred embodiment the transducer converts electrical energy into mechanical oscillations, and the rheology module comprises an electrical connector, whereby the electrical connector is provided as a single harness to provide electrical power and a signaling connection to the transducer and the at least one RF antenna unit. With the single harness, connection of the MR rheology module can easily be performed. The harness can comprise individual lines for power and signaling for the transducer and the RF antenna unit. The signaling line can be provided as bi-directional line for sending signals to the transducer and/or the RF antenna unit and receiving signals from the transducer and/or the RF antenna unit. Nevertheless, also independent signaling lines can be provided. Furthermore, multiple signaling lines can be provided for transmitting different kinds of signals. The harness can be further provided with a power line for the transducer and/or the RF antenna unit. A typical rheology module has four types of lines for the RF Antenna device, which are DC feed, RF signal, detune, and malfunction detection, and two lines for the transducer, which are a driving signal and a sensing line to monitor the performance of the transducer. Only one supply line for RF coils and transducer can be realized using a local semiconductor FET switch or amplifier inside the oscillator unit. A local amplifier can directly drive the transducer. Accordingly, B₀-compensation for the power line has to be performed only once.

According to a preferred embodiment the harness comprises at least one electrical line, which is connected to the transducer and the at least one RF antenna unit, and a filter unit provided in the electrical line for splitting signals received from the electrical line according to their frequency, whereby electrical signals on the electrical line are provided from the filter unit to the transducer and the RF antenna unit depending on their frequency. The electrical line can be a signaling line. Preferably, the electrical line is a combined signaling and power line. A typical MR signal has a frequency of some 10 MHz, power supply for the RF antenna device, in particular for preamplifiers associated to the RF antenna device, is a DC signal, and the transducer is driven by a signal having some 10Hz.

According to a preferred embodiment the harness comprises at least one digital signaling line, the rheology module comprises an AD/DA converter unit, which is connected to the digital signaling line, the transducer and the at least one RF antenna unit, and the AD/DA converter unit is adapted to perform a conversion and allocation of signals between the digital signaling line and the transducer and the at least one RF antenna unit. The signal conversion refers to AD/DA conversion. The allocation of the signals to the transducer and the RF antenna unit refers to multiplexing of signals from different RF coils to be transmitted, or to a separation of received signals, so that they can be provided to the correct recipient, i.e. the corresponding RF coil or the transducer. The digital line can be a bi-directional line, or a unidirectional line. The AD/DA converter is adapted to perform the required conversion. The AD conversion of the RF signal is preferably done on the RF antenna device, further preferred in the RF coil of the RF antenna device.

According to a preferred embodiment the digital signaling line is an optical signaling line. The optical signaling line enables high data transmission rates. Furthermore, the influence on the magnetic fields of the MR rheology imaging system is reduced compared to an electrical line, which generates a magnetic field when electrical signals are transmitted. The optical line is preferably a standard optical high-speed data connection.

According to a preferred embodiment the housing is flexible. The flexible housing facilitates the positioning of the rheology module. Furthermore, the rheology module can adapt to the form of the subject of interest, so that the RF antenna device can be in close contact thereto.

In another aspect of the present invention, the object is achieved by a rheology arrangement for use in a magnetic resonance (MR) rheology imaging system as defined in claim 8, comprising at least one RF antenna module comprising at least one RF antenna unit, and at least one rheology module as specified above, whereby the at least one RF antenna module and the at least one rheology module are interconnected. The modules can be arranged in any suitable way, e.g. as an array with multiple modules arranged in two directions, or as chain, where the modules are arranged in only one direction. Preferably, also an electrical connection between the modules is provided, so that the rheology arrangement can be easily connected. Further preferred, the rheology arrangement is provided with a single connector for electrically connecting all modules. The modules can be combined, i.e. attached to each other, outside the MR rheology imaging system. Preferably, a reversible connecting method is used for interconnecting the modules, e.g. zip or Velcro fastener. This allows easily adapting the applicator and coil array to the subject of interest and furthermore the ROI. The rheology arrangement can be tailored to fit the desired imaging/rheology measurement depending on the application, thus providing superior images.

According to a preferred embodiment the rheology arrangement is provided as a belt for application to the subject of interest. A belt can easily be positioned at the subject of interest and cover its entire circumference.

In a further aspect of the present invention, the object is achieved by a magnetic resonance (MR) rheology imaging system as defined in claim 10, comprising a main magnet for generating a static magnetic field, a magnetic gradient coil system for generating gradient magnetic fields superimposed to the static magnetic field, an examination space provided to position a subject of interest within, at least one radio frequency (RF) antenna device for applying an RF field to the examination space to excite nuclei of the subject of interest, a control unit for controlling the at least one RF antenna device, and at least one rheology module as specified above. The control unit is connected to the at least one rheology module and adapted to control the at least one rheology module, so that the MR rheology imaging system can autonomously introduce oscillations into the subject of interest and perform the required MR measurements. Preferably, information from RF antenna units installed on the MR imaging system is combined with information received from the rheology module.

According to a preferred embodiment the magnetic resonance (MR) rheology imaging system comprises a rheology arrangement as specified above, whereby the rheology arrangement comprises the at least one rheology module as specified above. Preferably, information from RF antenna units installed on the MR imaging system are combined with information received from the rheology module(s) and the RF antenna module(s).

### BRIEF DESCRIPTION OF THE DRAWINGS

These and other aspects of the invention will be apparent from and elucidated with reference to the embodiments described hereinafter. Such an embodiment does not necessarily represent the full scope of the invention, however, and reference is made therefore to the claims and herein for interpreting the scope of the invention.

In the drawings:
Fig. 1 is a schematic illustration of a part of an embodiment of a magnetic resonance (MR) imaging system in accordance with the invention,
Fig. 2 is a perspective view of a rheology module according to a first embodiment in accordance with the invention,
Fig. 3 is a top view of the rheology module according to Fig. 2,
Fig. 4 is a perspective view of a rheology module according to a second embodiment in accordance with the invention,
Fig. 5 is a schematic illustration of a rheology module according to a third embodiment in accordance with the invention,
Fig. 6 is a schematic illustration of a rheology module according to a fourth embodiment in accordance with the invention,
Fig. 7 is a schematic illustration of a rheology module according to a fifth embodiment in accordance with the invention, and
Fig. 8 is a schematic illustration of a rheology arrangement comprising a rheology module in accordance with the invention.

### DETAILED DESCRIPTION OF EMBODIMENTS

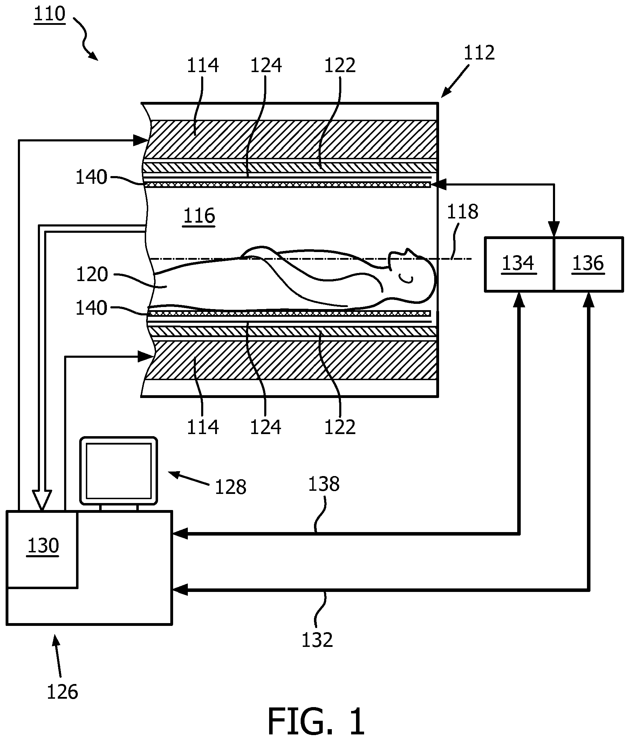

Fig. 1 shows a schematic illustration of a part of an embodiment of a magnetic resonance (MR) imaging system 110 comprising an MR scanner 112. The MR imaging system 110 includes a main magnet 114 provided for generating a static magnetic field. The main magnet 114 has a central bore that provides an examination space 116 around a center axis 118 for a subject of interest 120, usually a patient, to be positioned within. In this embodiment, the central bore and therefore the static magnetic field of the main magnet 114 has a horizontal orientation in accordance with the center axis 118. In an alternative embodiment, the orientation of the main magnet 114 can be different, e.g. to provide the static magnetic field with a vertical orientation. Further, the MR imaging system 110 comprises a magnetic gradient coil system 122 provided for generating gradient magnetic fields superimposed to the static magnetic field. The magnetic gradient coil system 122 is concentrically arranged within the bore of the main magnet 114, as known in the art.

Further, the MR imaging system 110 includes a radio frequency (RF) antenna device 140 designed as a whole-body coil having a tubular body. The RF antenna device 140 is provided for applying an RF magnetic field to the examination space 116 during RF transmit phases to excite nuclei of the subject of interest 120. The RF antenna device 140 is also provided to receive MR signal from the excited nuclei during RF receive phases. In a state of operation of the MR imaging system 110, RF transmit phases and RF receive phases are taking place in a consecutive manner. The RF antenna device 140 is arranged concentrically within the bore of the main magnet 114. As is known in the art, a cylindrical metal RF screen 124 is arranged concentrically between the magnetic gradient coil system 122 and the RF antenna device 140.

Moreover, the MR imaging system 110 comprises an MR image reconstruction unit 130 provided for reconstructing MR images from the acquired MR signals and an MR imaging system control unit 126 with a monitor unit 128 provided to control functions of the MR scanner 112, as is commonly known in the art. Control lines 132 are installed between the MR imaging system control unit 126 and an RF transmitter unit 134 that is provided to feed RF power of an MR radio frequency to the RF antenna device 140 via an RF switching unit 136 during the RF transmit phases. The RF switching unit 136 in turn is also controlled by the MR imaging system control unit 126, and another control line 138 is installed between the MR imaging system control unit 126 and the RF switching unit 136 to serve that purpose. During RF receive phase, the RF switching unit 136 directs the MR signals from the RF antenna device 140 to the MR image reconstruction unit 130 after pre-amplification.

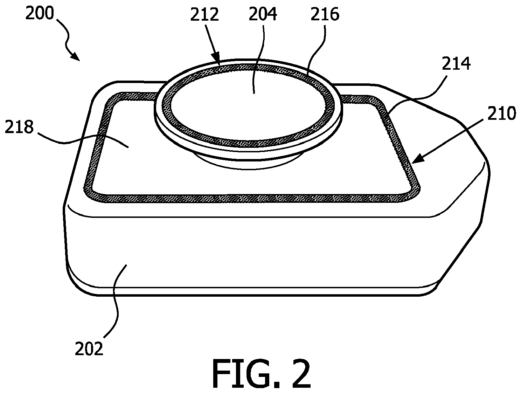

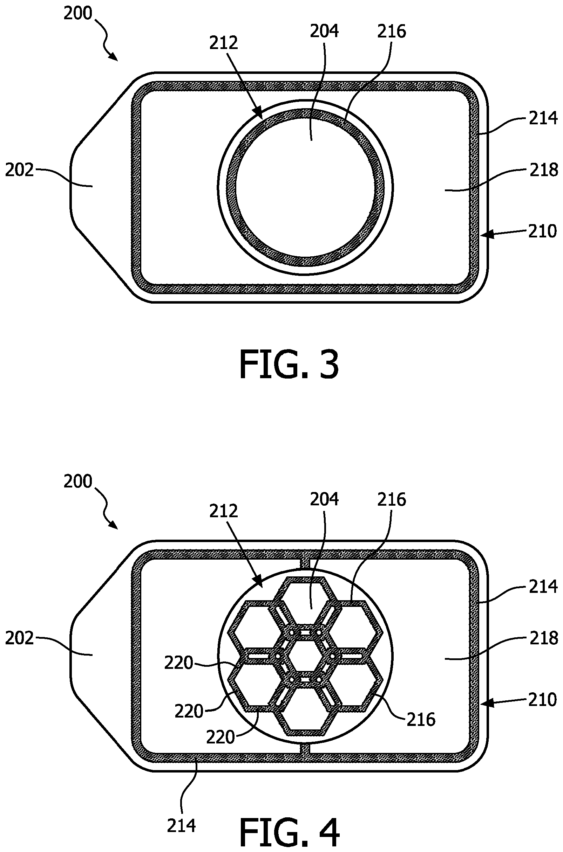

The MR imaging system 110 is provided as a MR rheology system comprising a rheology module 200, which is shown in Figs. 2 and 3, and which is adapted to introduce mechanical oscillations into the subject of interest 120.

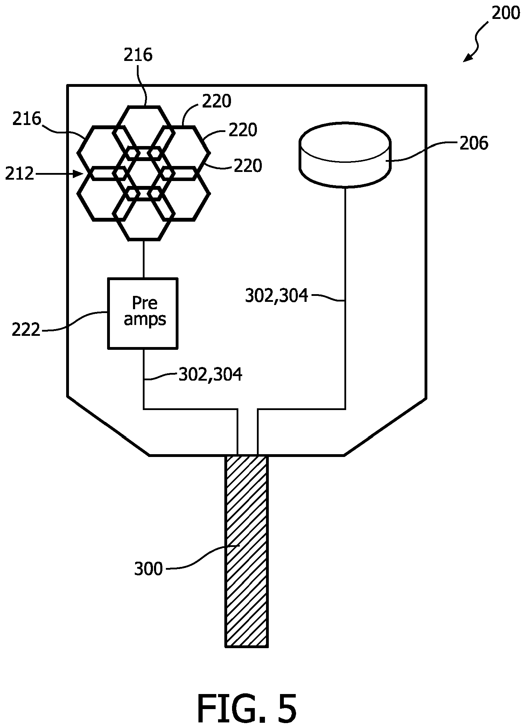

The rheology module 200 comprises a thin and flexible housing 202 and a mechanical oscillator unit 204, which is provided to be in contact with the subject of interest 120 in use. The oscillator unit 204 in this embodiment extends partially outside the housing 202 and is movable relative thereto. The rheology module 200 further comprises a transducer 206, which is indicated schematically in Figs. 5 to 7, for moving the oscillator unit 204. The transducer 206 in this embodiment is an electromechanical transducer, which converts electric energy into mechanical energy, i.e. into mechanical oscillations. The rheology module 200 further comprises a local, heavy mass, which is not shown in the figures and which is MR compatible, to provide mechanical inertia to keep the amplitude of the mechanical oscillations high. The local mass is exchangeable with respect to a desired application. In an alternative embodiment, the mass can be omitted in case the subject of interest is placed in such a manner that a table, on which the subject of interest can be placed, counters the rheology unit. Also, the MR rheology unit can be fixed to the patient bed or an inner wall of a bore of the MR rheology imaging system.

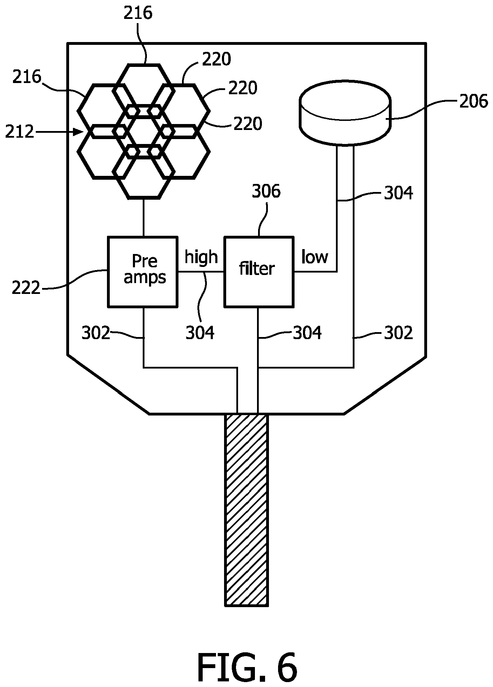

The rheology module 200 of this embodiment is provided with two RF antenna units 210, 212, which are respectively located at the housing 202 and the oscillator unit 204. Each RF antenna unit 210, 212 in this embodiment comprises one RF coil 214, 216, respectively. The RF coil 214 of the RF antenna unit 210 located at the housing 202 is provided at an upper face 218 of the housing 202, through which the oscillator unit 204 is connected to the transducer 206. The RF coil 214 is provided at an upper surface of the housing 202, i.e. at an upper surface of the upper face 218, and has a rectangular shape extending along the sides of the upper face 218. Accordingly, the RF coil 214 surrounds the oscillator unit 204. The RF coil 216 of the RF antenna unit 212 located at the oscillator unit 204 has a circular shape and is provided at an upper surface of the oscillator unit 204. The rheology module 200 also comprises pre-amplifiers 220, which are provided within the housing 202 for driving the RF coils 214, 216, as indicated in Figs. 5 and 6.

The control unit 126 of the MR rheology imaging system 110 is connected to the rheology module 200 and adapted to control the rheology module 200, so that the MR rheology imaging system 110 can autonomously introduce mechanical oscillations into the subject of interest 120 and perform MR rheology imaging operations. A physical connection between the rheology module 200 and the control unit 126 is described in detail below.

A second embodiment of the rheology module 200 is shown in Fig. 4. The rheology module 200 is similar to the rheology module 200 of the first embodiment, so that only the differences will be described in detail.

The rheology module 200 of the second embodiment differs from that of the first embodiment in the structure of the RF antenna units 210, 212. According to the second embodiment, the RF antenna unit 210 located at the housing 202 comprises two rectangular RF coils 214, which are provided at an upper face 218 of the housing 202 as described above. Each RF coil 214 extends over half the area of the upper face 218. The RF antenna unit 212 located at the oscillator unit 204 comprises a set of seven individual RF coils 216, each of which has an essentially circular shape formed by six linear segments 220. The RF coils 216 of the RF antenna unit 216 are arranged in an array overlapping with adjacent RF coils 216. The rheology module 200 also comprises pre-amplifiers 222, which are provided within the housing 202 for driving the RF coils 214, 216, as indicated in Figs. 5 and 6. Although the pre-amplifiers 222 are indicated in Figs. 5 and 6 as a single box, each RF coil 214, 216 has one pre-amplifier 222 associated thereto.

The control unit 126 of the MR rheology imaging system 110 is connected to the rheology module 200 and adapted to control the rheology module 200, so that the MR rheology imaging system 110 can autonomously introduce oscillations into the subject of interest 120 and perform MR rheology imaging operations. The physical connection between the rheology module 200 and the control unit 126 is described in detail below.

Fig. 5 shows a rheology module 200 according to a third embodiment with a physical connection. By way of example, the physical connection is illustrated based on the rheology module 200 of the second embodiment, as indicated by the RF antenna device 212 located in the oscillator unit 204 having multiple RF coils 216. Nevertheless, the connection can be realized without general modifications for other rheology modules 200, e.g. that of the first embodiment.

The rheology module 200 according to the third embodiment comprises an electrical connector 300, which is provided as a single harness 300. The electrical connector 300 provides electrical power and a signaling connection to the transducer 206 and the RF coils 214, 216 of the RF antenna units 210, 212. The electrical connector 300 comprises an individual power line 302 and signaling lines 304 for the transducer 206 and the RF antenna units 210, 212. The power line 302 and the signaling lines 304 are indicated by a single line in Fig. 5. In particular, the pre-amplifiers 222 are connected by four lines 302, 304, which are DC feed as power line 302, as well as RF signal, detune, and malfunction detection as signaling lines 304. The transducer 206 is connected by two lines 302, 304, which are a driving signal and a sensing line to monitor the performance of the transducer 206. The signaling lines 304 are provided as bi-directional lines for sending signals to the transducer 206 and the RF antenna units 210, 212 and receiving signals from the transducer 206 and the RF antenna units 210, 212. The power lines 302 are B₀-compensated.

Fig. 6 shows a rheology module 200 according to a fourth embodiment with a physical connection. The rheology module 200 of the fourth embodiment only differs in the connection of its RF antenna units 210, 212 and transducer 206 to a harness 300 from the rheology module 200 of the third embodiment. Accordingly, only the differences between these rheology modules 200 will be discussed.

The rheology module 200 according to the fourth embodiment comprises an electrical connector 300, which is provided as a single harness 300. The harness 300 comprises power lines 304, which are directly connected to the transducer 206 and pre-amplifiers 222 of the RF antenna units 210, 212. The harness 300 further comprises a signaling line 304, which is connected to a filter unit 306 of the rheology module 200. The filter unit 306 is adapted for splitting signals received from the signaling line 304 according to their frequency.

Electrical signals on the signaling line 304 are provided from the filter unit 306 to the transducer 206 and the RF antenna units 210, 212 depending on their frequency. MR signals having a typical frequency of some 10 MHz are provided to the RF antenna units 210, 212, and a driving signal having some 10Hz is provided to the transducer 206. A threshold for splitting the signals is defined between these frequencies.

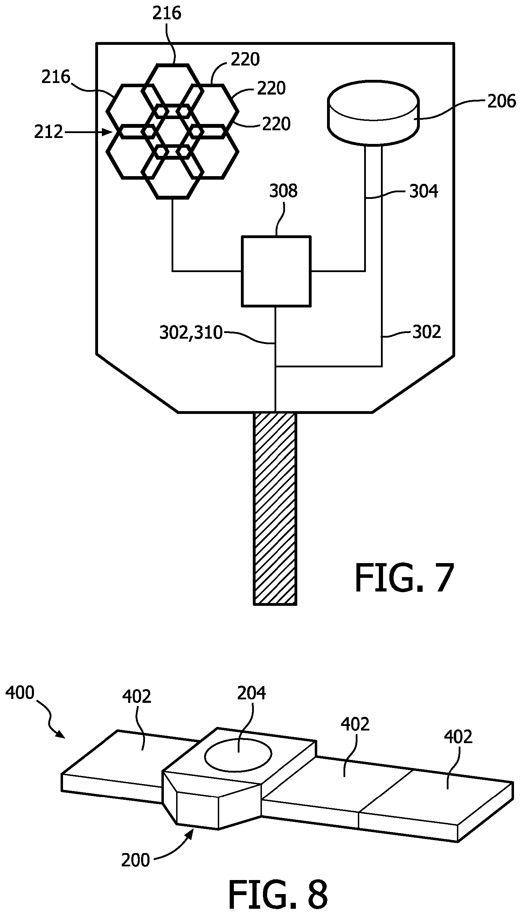

Fig. 7 shows a rheology module 200 according to a fifth embodiment with a physical connection. The rheology module 200 of the fifth embodiment only differs in the connection of its RF antenna units 210, 212 and transducer 206 to a harness 300 from the rheology module 200 of the third embodiment. Accordingly, only the differences between these rheology modules 200 will be discussed.

The rheology module 200 according to the fifth embodiment comprises an electrical connector 300, which is provided as a single harness. The rheology module 200 comprises an AD/DA converter unit 308, which is in this embodiment integrally provided with pre-amplifiers 222 in a driving box 308. The driving box 308 is connected to a digital signaling line 310 and a power line 302, whereby the power line 302 provides power for the pre-amplifiers 222. A transducer 206 is connected with a separate power line 302 of the harness 300. The signaling line 310 is used for signaling to/and from the transducer 206 and the preamplifiers 222 in the driving box 308. An analog signaling line 304 is provided between the transducer 206 and the AD/DA converter unit 308. The AD/DA converter unit 308 performs an AD/DA conversion. Additionally, an allocation of signals between the digital signaling line and the transducer 206 and the RF antenna unit, i.e. the pre-amplifiers 222 is performed, so that all signals are multiplexed on the digital signaling line 310. The digital signaling line 310 is a bi-directional line.

In an alternative embodiment the digital signaling line 310 in the harness 300 is an optical digital signaling line.

A sixth embodiment refers to a rheology arrangement 400 for use in a magnetic resonance (MR) rheology imaging system 110, which is shown in Fig. 8. The rheology arrangement 400 comprises three RF antenna modules 402, which comprises a RF antenna unit as described above in respect to the rheology modules 200, and one rheology module 200 as specified above. The modules 200, 402 are interconnected and arranged in a chain, so that the rheology arrangement 400 can be used as a belt for application to the subject of interest 120. The modules 200, 402 are attached to each other by Velcro fasteners, which are not explicitly shown in Fig. 8. In an alternative embodiment, the modules 200, 402 comprise electrical connectors for connecting to adjacent modules 200, 402, and the rheology arrangement 400 comprises a single connector for electrically connecting all modules 200, 402 to the MR rheology imaging system 110.

According to a modified embodiment, the magnetic resonance (MR) rheology imaging system 110 comprises a rheology arrangement 400, whereby the rheology arrangement 400 comprises the at least one rheology module 200.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

### REFERENCE SYMBOL LIST

- 110: magnetic resonance (MR) imaging system
- 112: magnetic resonance (MR) scanner
- 114: main magnet
- 116: RF examination space
- 118: center axis
- 120: subject of interest
- 122: magnetic gradient coil system
- 124: RF screen
- 126: MR imaging system control unit
- 128: monitor unit
- 130: MR image reconstruction unit
- 132: control line
- 134: RF transmitter unit
- 136: RF switching unit
- 138: control line
- 140: radio frequency (RF) antenna device
- 200: rheology module
- 202: housing
- 204: oscillator unit
- 206: transducer
- 210: RF antenna unit (at housing)
- 212: RF antenna unit (at oscillator)
- 214: RF coil (at housing)
- 216: RF coil (at oscillator)
- 218: upper face
- 220: linear segment
- 222: pre-amplifier
- 300: electrical connector, harness
- 302: power line
- 304: signaling line
- 306: filter unit
- 308: AD/DA converter unit
- 310: digital signaling line
- 400: rheology arrangement
- 402: RF antenna module

## Claims

1. A rheology module (200) for use in a magnetic resonance (MR) rheology imaging system (110), whereby the rheology module (200) is adapted to introduce mechanical oscillations into a subject of interest (120), comprising
a housing (202),
a mechanical oscillator unit (204), which is arranged at least partially inside the housing, extends at least partially outside the housing (202) and is movable as a whole relative to the housing (202), and
a transducer (206) for moving the oscillator unit (204),
whereby
the rheology module (200) comprises at least two radio frequency (RF) antenna units (210, 212), each of which comprises at least one RF coil (214, 216),
whereby
a first of the at least two RF antenna units (210) is located on or in the housing (202), and
**characterized in that** a second of the at least two RF antenna units (212) is located on or in the oscillator unit (204).

2. The rheology module (200) according to claim 1, whereby the second RF antenna unit (212) comprises a set of multiple RF coils (216).

3. The rheology module (200) according to any preceding claim, whereby
the transducer (206) converts electrical energy into mechanical oscillations,
and
the rheology module (200) comprises an electrical connector (300),whereby
the electrical connector (300) is provided as a single harness to provide electrical power and a signaling connection to the transducer (206) and the at least two RF antenna units (210, 212).

4. The rheology module (200) according to claim 3, whereby the harness (300) comprises at least one electrical line (302, 304), which is connected to the transducer (206) and the at least two RF antenna units (210, 212), and
a filter unit (306) is provided in the electrical line (302, 304) for splitting signals received from the electrical line (302, 304) according to their frequency,
whereby
electrical signals on the electrical line (302, 304) are provided from the filter unit (306) to the transducer (206) and the at least two RF antenna units (210, 212) depending on their frequency.

5. The rheology module (200) according to claim 3 or 4, whereby
the harness (300) comprises at least one digital signaling line (310),
the rheology module (200) comprises an AD/DA converter unit (308), which is connected to the digital signaling line (310), the transducer (206) and the at least two RF antenna units (210, 212), and the AD/DA converter unit (308) is adapted to perform a conversion and allocation of signals between the digital signaling line (310) and the transducer (206) and the at least two RF antenna units (210, 212).

6. The rheology module (200) according to claim 5, whereby
the digital signaling line (310) is an optical signaling line.

7. The rheology module (200) according to any preceding claim, whereby
the housing (202) is flexible.

8. A rheology arrangement (400) for use in a magnetic resonance (MR) rheology imaging system (110), comprising
at least one RF antenna module (402) comprising at least one RF antenna unit,
and
at least one rheology module (200) according to any of claims 1 to 7, whereby
the at least one RF antenna module (402) and the at least one rheology module (200) are interconnected.

9. The rheology arrangement (400) according to claim 8, whereby
the rheology arrangement (400) is provided as a belt for application to the subject of interest (120).

10. A magnetic resonance (MR) rheology imaging system (110), comprising
a main magnet (114) for generating a static magnetic field,
a magnetic gradient coil system (122) for generating gradient magnetic fields superimposed to the static magnetic field,
an examination space (116) provided to position a subject of interest (120) within,
at least one radio frequency (RF) antenna device (140) for applying an RF field to the examination space (116) to excite nuclei of the subject of interest (120),
a control unit (126) for controlling the at least one RF antenna device (140),
and
at least one rheology module (200) according to any of claims 1 to 7.

11. The magnetic resonance (MR) rheology imaging system (110) according to claim 10, comprising a rheology arrangement (400) according to claim 8 or 9, whereby
the rheology arrangement (400) comprises the at least one rheology module (200) according to any of preceding claims 1 to 7.

## Patentansprüche

1. Rheologiemodul (200) zur Verwendung in einem Magnetresonanz (MR)-Rheologie-Bildgebungssystem (110), wobei das Rheologiemodul (200) angepasst ist, um mechanische Schwingungen in ein Subjekt von Interesse (120) einzuführen, umfassend
ein Gehäuse (202),
eine mechanische Oszillatoreinheit (204), die zumindest teilweise innerhalb des Gehäuses angeordnet ist, sich zumindest teilweise außerhalb des Gehäuses (202) erstreckt und als Ganzes relativ zu dem Gehäuse (202) beweglich ist, und
einen Wandler (206) zum Bewegen der Oszillatoreinheit (204),
wobei
das Rheologiemodul (200) mindestens zwei Hochfrequenz(HF)-Antenneneinheiten (210, 212) umfasst, von denen jede mindestens eine HF-Spule (214, 216) umfasst;
wobei
eine erste der mindestens zwei HF-Antenneneinheiten (210) sich am oder im Gehäuse (202) befindet, und **dadurch gekennzeichnet, dass**
eine zweite der mindestens zwei HF-Antenneneinheiten (212) sich an oder in der Oszillatoreinheit (204) befindet.

2. Rheologiemodul (200) nach Anspruch 1, wobei
die zweite HF-Antenneneinheit (212) einen Satz mehrerer HF-Spulen (216) umfasst.

3. Rheologiemodul (200) gemäß einem vorhergehenden Anspruch, wobei
der Wandler (206) elektrische Energie in mechanische Schwingungen umwandelt, und
das Rheologiemodul (200) einen elektrischen Verbinder (300) umfasst, wobei
der elektrische Verbinder (300) als ein einziger Kabelbaum vorgesehen ist, um elektrische Energie und eine Signalverbindung zu dem Wandler (206) und den mindestens zwei HF-Antenneneinheiten (210, 212) bereitzustellen.

4. Rheologiemodul (200) nach Anspruch 3, wobei
der Kabelbaum (300) mindestens eine elektrische Leitung (302, 304) umfasst, die mit dem Wandler (206) und den mindestens zwei HF-Antenneneinheiten (210, 212) verbunden ist, und
in der elektrischen Leitung (302, 304) eine Filtereinheit (306) vorgesehen ist, um die von der elektrischen Leitung (302, 304) empfangenen Signale entsprechend ihrer Frequenz aufzuteilen,
wobei
elektrische Signale auf der elektrischen Leitung (302, 304) von der Filtereinheit (306) zu dem Wandler (206) und den mindestens zwei HF-Antenneneinheiten (210, 212) abhängig von ihrer Frequenz geliefert werden.

5. Rheologiemodul (200) nach Anspruch 3 oder 4, wobei
der Kabelbaum (300) mindestens eine digitale Signalleitung (310) umfasst,
das Rheologiemodul (200) eine AD/DA-Wandlereinheit (308) umfasst, die mit der digitalen Signalleitung (310), dem Wandler (206) und den mindestens zwei HF-Antenneneinheiten (210, 212) verbunden ist, und
die AD/DA-Wandlereinheit (308) angepasst ist, um eine Umwandlung und Zuteilung von Signalen zwischen der digitalen Signalleitung (310) und dem Wandler (206) und den mindestens zwei HF-Antenneneinheiten (210, 212) durchzuführen.

6. Rheologiemodul (200) nach Anspruch 5, wobei
die digitale Signalleitung (310) eine optische Signalleitung ist.

7. Rheologiemodul (200) nach einem vorhergehenden Anspruch, wobei
das Gehäuse (202) flexibel ist.

8. Rheologieanordnung (400) zur Verwendung in einem Magnetresonanz (MR)-Rheologie-Bildgebungssystem (110), umfassend
mindestens ein HF-Antennenmodul (402), das mindestens eine HF-Antenneneinheit umfasst, und
mindestens ein Rheologiemodul (200) nach einem der Ansprüche 1 bis 7, wobei
das mindestens eine HF-Antennenmodul (402) und das mindestens eine Rheologiemodul (200) miteinander verbunden sind.

9. Rheologieanordnung (400) nach Anspruch 8, wobei
die Rheologieanordnung (400) als Gürtel zur Anwendung auf das Subjekt von Interesse (120) vorgesehen ist.

10. Magnetresonanz (MR)-Rheologie-Bildgebungssystem (110), umfassend
einen Hauptmagneten (114) zum Erzeugen eines statischen Magnetfeldes,
ein magnetisches Gradientenspulensystem (122) zum Erzeugen von Gradientenmagnetfeldern, die dem statischen Magnetfeld überlagert sind,
einen Untersuchungsraum (116), der vorgesehen ist, um ein Subjekt von Interesse (120) darin zu positionieren,
mindestens eine Hochfrequenzantennenvorrichtung RF (140) zum Anlegen eines HF-Feldes an den Untersuchungsraum (116), um Kerne des interessierenden Subjekts (120) anzuregen;
eine Steuereinheit (126) zum Steuern der mindestens einen HF-Antennenvorrichtung (140), und
mindestens ein Rheologiemodul (200) nach einem der Ansprüche 1 bis 7.

11. Magnetresonanz (MR)-Rheologie-Bildgebungssystem (110) nach Anspruch 10, umfassend
eine Rheologieanordnung (400) nach Anspruch 8 oder 9,
wobei,
die Rheologieanordnung (400) das mindestens eine Rheologiemodul (200) gemäß einem der vorhergehenden Ansprüche 1 bis 7 umfasst.

## Revendications

1. Module de rhéologie (200) destiné à être utilisé dans un système d'imagerie de rhéologie par résonance magnétique (RM) (110), dans lequel le module de rhéologie (200) est adapté pour introduire des oscillations mécaniques dans un sujet d'intérêt (120), comprenant
un boîtier (202),
une unité d'oscillateur mécanique (204), qui est agencée au moins partiellement à l'intérieur du boîtier, s'étend au moins partiellement à l'extérieur du boîtier (202) et est mobile dans son ensemble par rapport au boîtier (202), et
un transducteur (206) pour déplacer l'unité d'oscillateur (204),
où
le module de rhéologie (200) comprend au moins deux unités d'antennes de radiofréquence (RF) (210, 212), dont chacune comprend au moins une bobine RF (214, 216) ,
où
une première des au moins deux unités d'antennes RF (210) est située sur ou dans le boîtier (202), et **caractérisé en ce que**
une seconde des au moins deux unités d'antennes RF (212) est située sur ou dans l'unité d'oscillateur (204) .

2. Module de rhéologie (200) selon la revendication 1, dans lequel
la seconde unité d'antenne RF (212) comprend un ensemble de plusieurs bobines RF (216).

3. Module de rhéologie (200) selon l'une quelconque des revendications précédentes, dans lequel
le transducteur (206) convertit l'énergie électrique en oscillations mécaniques, et
le module de rhéologie (200) comprend un connecteur électrique (300), où
le connecteur électrique (300) est prévu sous la forme d'un câblage unique pour fournir une alimentation électrique et une connexion de signalisation au transducteur (206) et aux au moins deux unités d'antennes RF (210, 212) .

4. Module de rhéologie (200) selon la revendication 3, dans lequel
le câblage (300) comprend au moins une ligne électrique (302, 304), qui est connectée au transducteur (206) et aux au moins deux unités d'antennes RF (210, 212), et
une unité de filtre (306) est prévue dans la ligne électrique (302, 304) pour séparer les signaux reçus de la ligne électrique (302, 304) en fonction de leur fréquence,
où
des signaux électriques sur la ligne électrique (302, 304) sont fournis par l'unité de filtre (306) au transducteur (206) et aux au moins deux unités d'antennes RF (210, 212) en fonction de leur fréquence.

5. Module de rhéologie (200) selon la revendication 3 ou 4, dans lequel
le câblage (300) comprend au moins une ligne de signalisation numérique (310),
le module de rhéologie (200) comprend une unité de conversion AD/DA (308), qui est connectée à la ligne de signalisation numérique (310), au transducteur (206) et aux au moins deux unités d'antennes RF (210, 212), et
l'unité de conversion AD/DA (308) est adaptée pour effectuer une conversion et une attribution de signaux entre la ligne de signalisation numérique (310) et le transducteur (206) et les au moins deux unités d'antennes RF (210, 212) .

6. Module de rhéologie (200) selon la revendication 5, dans lequel
la ligne de signalisation numérique (310) est une ligne de signalisation optique.

7. Module de rhéologie (200) selon l'une quelconque des revendications précédentes, dans lequel
le boîtier (202) est flexible.

8. Agencement de rhéologie (400) destiné à être utilisé dans un système d'imagerie de rhéologie par résonance magnétique (RM) (110) comprenant
au moins un module d'antenne RF (402) comprenant au moins une unité d'antenne RF, et
au moins un module de rhéologie (200) selon l'une quelconque des revendications 1 à 7, où
l'au moins un module d'antenne RF (402) et l'au moins un module de rhéologie (200) sont interconnectés.

9. Agencement de rhéologie (400) selon la revendication 8, dans lequel
l'agencement de rhéologie (400) est prévu sous la forme d'une ceinture à appliquer sur le sujet d'intérêt (120) .

10. Système d'imagerie de rhéologie par résonance magnétique (RM) (110), comprenant
un aimant principal (114) pour générer un champ magnétique statique,
un système de bobine à gradient magnétique (122) pour générer des champs magnétiques à gradient superposés au champ magnétique statique,
un espace d'examen (116) prévu pour y placer un sujet d'intérêt (120),
au moins un dispositif d'antenne de radiofréquence (RF) (140) pour appliquer un champ RF à l'espace d'examen (116) pour exciter les noyaux du sujet d'intérêt (120),
une unité de commande (126) pour commander l'au moins un dispositif d'antenne RF (140), et
au moins un module de rhéologie (200) selon l'une quelconque des revendications 1 à 7.

11. Système d'imagerie de rhéologie par résonance magnétique (RM) (110) selon la revendication 10, comprenant
un agencement de rhéologie (400) selon la revendication 8 ou 9,
où
l'agencement de rhéologie (400) comprend l'au moins un module de rhéologie (200) selon l'une quelconque des revendications 1 à 7 précédentes.
